# EUROPEAN PATENT APPLICATION

(11) **EP 4 537 690 A1**
(43) Date of publication of application: **16.04.2025**
(21) Application number: 23818804.9
(22) Date of filing: 31.03.2023
(51) Int. Cl.: A24F 40/50, A24F 40/10

(54) **ATOMIZATION CONTROL METHOD AND ELECTRONIC ATOMIZER**

(30) Priority: 06.06.2022 CN 202210627507
(71) Applicant: Hainan Moore Brothers Technology Co., Ltd., Hainan 571900 (CN)
(72) Inventor: LI, Huanxi, Chengmai 571900 (CN); CHU, Qingchen, Chengmai 571900 (CN); ZHOU, Hongming, Chengmai 571900 (CN)
(74) Representative: Michalski Hüttermann & Partner Patentanwälte mbB
(86) International application number: PCT/CN2023/085472
(87) International publication number: WO 2023/236636

(57) **Abstract**

This application disclose an atomization control method and an electronic atomizer. The atomization control method includes the following steps: obtaining a puffing signal and heating to atomize an aerosol-generating substrate strip (30) in response to the puffing signal; obtaining a real-time count of puffs, and releasing to update the aerosol-generating substrate strip (30) in case that the real-time count of puffs is equal to a preset number of puffs.

## Description

### CROSS-REFERENCE TO RELATED APPLICATIONS

This application claims priority to Chinese Patent Application No. 202210627507.4, filed with CNIPA on June 6, 2022 and entitled "ATOMIZATION CONTROL METHOD AND ELECTRONIC ATOMIZER", the entire contents of which are incorporated herein by reference.

### TECHNICAL FIELD

The present disclosure relates to the field of atomization technologies, and in particular, to an atomization control method and an electronic atomizer.

### BACKGROUND

Aerosols are colloidal dispersions formed by solid or liquid particles dispersed and suspended in a gaseous medium. Aerosols can be inhaled by the human body through the respiratory system, providing a new alternative intake method for users. An atomizer is a device that atomizes a stored atomizable aerosol-generating substrate to form aerosols through heating or ultrasonic means. The atomizable aerosol-generating substrate includes e-liquids containing nicotine, medical drugs, skincare lotions, etc. Atomizing these aerosol-generating substrates can provide inhalable aerosols to users, offering an alternative to conventional product forms and intake methods.

However, current electronic atomizers require preheating of the aerosol-generating substrate before use, which leads to issues such as long preheating time, slow heating speed, and poor vaping consistency, making them inconvenient for use and hindering the wider adoption of electronic atomizers.

### SUMMARY

According to various embodiments of the present disclosure, an atomization control method and an electronic atomizer are provided.

An atomization control method includes:
obtaining a puffing signal, heating to atomize an aerosol-generating substrate strip in response to the puffing signal; and
obtaining a real-time count of puffs, and releasing to update the aerosol-generating substrate strip in case that the real-time count of puffs is equal to a preset number of puffs.

In an embodiment, the atomization control method further includes:
obtaining a real-time released length of the aerosol-generating substrate strip; and
stopping updating the aerosol-generating substrate strip in case that the real-time released length is equal to a first preset length.

In an embodiment, the electronic atomizer includes a rotating member that rotates along with the release of the aerosol-generating substrate strip, and obtaining the real-time released length of the aerosol-generating substrate strip specifically includes:
obtaining a rotation angle or a number of rotations of the rotating member; and
obtaining the real-time released length of the aerosol-generating substrate strip based on the rotation angle or the number of rotations.

In an embodiment, the electronic atomizer includes a feed motor for releasing the aerosol-generating substrate strip. The feed motor has a motor encoder. Obtaining the real-time released length of the aerosol-generating substrate strip specifically includes:
obtaining a number of rotations of the motor encoder of the feed motor; and
obtaining the real-time released length of the aerosol-generating substrate strip based on the number of rotations of the motor encoder.

In an embodiment, the aerosol-generating substrate strip is provided with detection markers arranged at intervals along a length direction of the aerosol-generating substrate strip, and obtaining the real-time released length of the aerosol-generating substrate strip specifically includes:
obtaining a number of detection markers on the aerosol-generating substrate strip; and
obtaining the real-time released length of the aerosol-generating substrate strip based on the number of detection markers.

In an embodiment, before obtaining the puffing signal and heating to atomize the aerosol-generating substrate strip in response to the puffing signal, the atomization control method further includes:
obtaining a start signal, and releasing a second preset length of the aerosol-generating substrate strip in response to the start signal.

In an embodiment, the atomization control method further includes:
obtaining a stop signal, and releasing a third preset length of the aerosol-generating substrate strip in response to the stop signal.

An electronic atomizer includes:
a main control module;
a start and stop signal input module which is in communication connection with the main control module and is configured to receive a start signal and feed the start signal back to the main control module;
a drive module in communication connection with the main control module, the drive module being configured to release, under control of the main control module, the aerosol-generating substrate strip for updating; and
an atomization module in communication connection with the main control module, the atomization module being configured to heat to atomize the aerosol-generating substrate strip under control of the main control module.

In an embodiment, the electronic atomizer further includes a feed detection module. The feed detection module is in communication connection with the main control module and is configured to obtain a real-time released length of the aerosol-generating substrate strip and feed the real-time released length back to the main control module.

In an embodiment, the electronic atomizer further includes a status indication module. The status indication module is in communication connection with the main control module and is configured to indicate an operating status of the electronic atomizer.

In an embodiment, the electronic atomizer further includes a substrate container and an installation detection module. The substrate container is configured to accommodate the aerosol-generating substrate strip, and the installation detection module is configured to detect whether the substrate container exists in the electronic atomizer.

In an embodiment, the electronic atomizer further includes a residual detection module, which is configured to detect a residual amount of the aerosol-generating substrate strip.

The details of one or more embodiments of the present disclosure are set forth in the accompanying drawings and the description below. Other features, objects, and advantages of the disclosure will be apparent from the description, drawings, and claims.

### BRIEF DESCRIPTION OF THE DRAWINGS

In order to illustrate the technical solutions in the embodiments of the present disclosure or prior art more clearly, the accompanying drawings used in the description of the embodiments or prior art will be briefly introduced below. Apparently, the accompanying drawings in the following description are only some embodiments of the present disclosure. For those of ordinary skill in the art, other drawings can also be derived from these drawings without creative effort.
FIG. 1 is a schematic diagram showing modules of an electronic atomizer according to an embodiment of the present disclosure.
FIG. 2 is a schematic diagram of a drive module of an electronic atomizer according to an embodiment of the present disclosure.
FIG. 3 is a schematic diagram of a drive module of an electronic atomizer according to another embodiment of the present disclosure.
FIG. 4 is a flowchart of an atomization control method according to an embodiment of the present disclosure.
FIG. 5 is a flowchart of an atomization control method according to an embodiment of the present disclosure.

### Description of reference numerals:

11. main control module; 12. start and stop signal input module; 13. drive module; 131. release reel; 132. retrieve reel; 133. conveyor roller; 135. release reel; 136. driving feed roller group; 137. roller shaft group; 14. atomization module; 16. feed detection module; 162. rotating member; 17. status indication module; 18. installation detection module; 19. residual detection module; 30. retrieve cavity; 30. aerosol-generating substrate strip.

### DETAILED DESCRIPTION OF THE EMBODIMENTS

To make the purpose, features, and advantages of the present disclosure more obvious and understandable, detailed explanations of specific embodiments of the present disclosure are provided below in conjunction with the accompanying drawings. Many specific details are described below to facilitate a full understanding of the present disclosure. However, the present disclosure can be implemented in many other ways different from those described herein. Those skilled in the art can make similar improvements without departing from the essence of the present disclosure. Therefore, the present disclosure is not limited to the specific exemplary embodiments disclosed below.

In the description of the present disclosure, it should be understood that terms such as "center", "longitudinal", "lateral", "length", "width", "thickness", "upper", "lower", "front", "back", "left", "right", "vertical", "horizontal", "top", "bottom", "inner", "outer", "clockwise", "counterclockwise", "axial", "radial", "circumferential", etc., indicate orientations or positional relationships as shown in the drawings, and are only used for the purpose of describing the present disclosure and simplifying the description, not indicating or implying that the described devices or components must have specific orientations, be constructed and operated in specific orientations. Therefore, these terms should not be interpreted as limitations on the present disclosure.

In addition, the terms "first" and "second" are used only for descriptive purposes and should not be construed as indicating or implying relative importance and specific quantities of the described technical features. Therefore, features described with "first", "second", etc., may include at least one of those features, whether explicitly stated or implied. In the description of the present disclosure, the term "multiple" means at least two, such as two, three, etc., unless otherwise specifically limited.

In the present disclosure, unless otherwise explicitly specified and limited, terms such as "installation", "connection", "coupling", "fixing", etc., should be interpreted in a broad sense. For example, it may refer to fixed connections, detachable connections, or integration. It may be mechanical connections or electrical connections. It may be direct connections or indirect connections through an intermedium. It may be internal communication between two components or the interaction relationship between two components, unless otherwise explicitly specified. Ordinary skilled persons in the art can understand the specific meanings of the above terms in the present disclosure based on specific circumstances.

In the present disclosure, unless otherwise explicitly specified and limited, the expression a first feature being "on" or "under" a second feature may mean that the first feature is in direct contact with the second feature, or the first feature is in indirect contact with the second feature via an intermediate medium. Furthermore, the expression the first feature being "over", "above" and "on top of" the second feature may mean that the first feature is directly above or obliquely above the second feature, or only means that the level of the first feature is higher than that of the second feature. The expression the first feature being "below", "underneath" or "under" the second feature may mean that the first feature is directly underneath or obliquely underneath the second feature, or only means that the level of the first feature is lower than that of the second feature.

It should be noted that when a component is referred to as being "fixed to" or "set on" another component, it may be directly on the other component or there may also be an intermedium component. If a component is considered to be "connected to" another component, it may be directly connected to the other component or there may also be an intermedium component. The terms 'vertical', 'horizontal', 'above', 'below', 'left', 'right', and similar expressions used in the present disclosure are for illustrative purposes only and do not imply that they are the only embodiments.

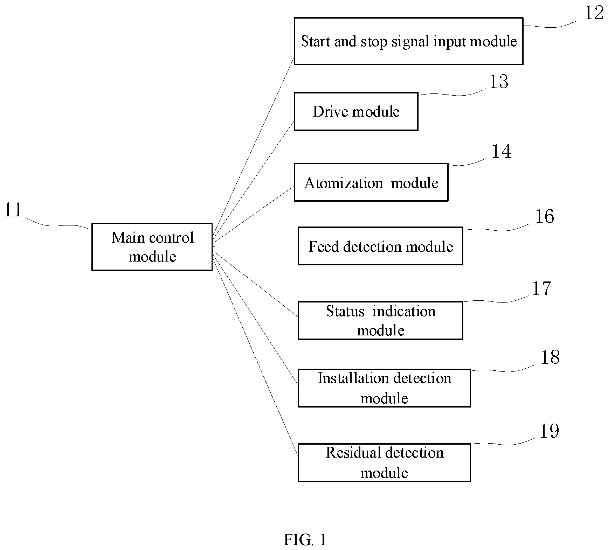

Referring to FIG. 1, an embodiment of the present disclosure provides an electronic atomizer for heating to atomize an aerosol-generating substrate strip 30 to generate aerosols for a user to inhale.

The electronic atomizer includes a main housing, and a main control module 11, a start and stop signal input module 12, a drive module 13, an atomization module 14, and a power supply module that are received in the main housing. The start and stop signal input module 12, the drive module 13, and the atomization module 14 are in communication connection with the main control module 11, respectively. The start signal module is configured to receive start signals or stop signals and feed them back to the main control module 11. The drive module 13 is configured to release to update the aerosol-generating substrate strip 30 under the control of the main control module 11. The atomization module 14 is configured to heat to atomize the aerosol-generating substrate strip 30 under the control of the main control module 11 to generate aerosols. The power supply module is electrically connected to the main control module 11, the start and stop signal input module 12, the drive module 13, and the atomization module 14, and configured to supply power for the main control module 11, the start and stop signal input module 12, the drive module 13, and the atomization module 14.

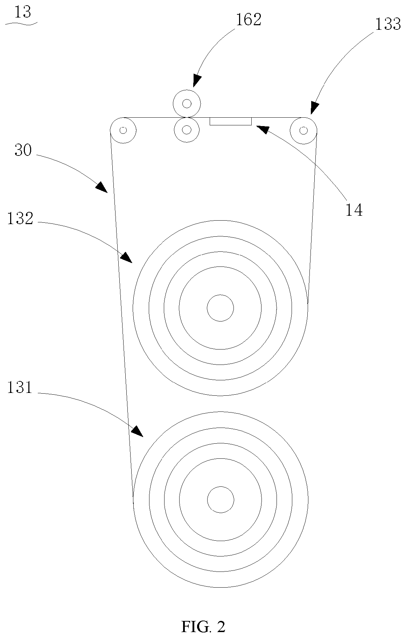

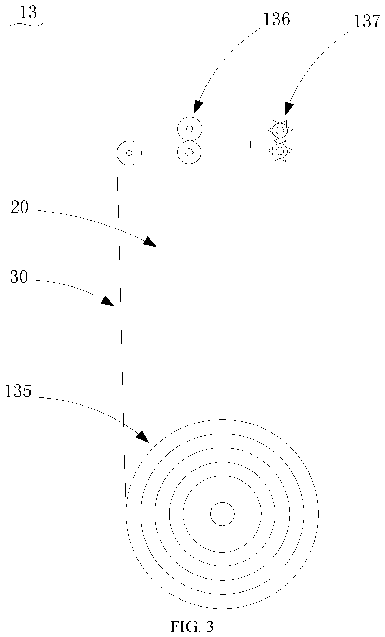

As shown in FIG. 2 and FIG. 3, specifically, the aerosol-generating substrate strip 30 is in a form of a long strip, which can be wound layer by layer in the circumferential direction to form a substrate roll accommodated in a substrate container, and the substrate container is detachably accommodated in the main housing. Compared to aerosol-generating substrates in the form of columns or other shapes, the aerosol-generating substrate strip 30 is very thin, which allows its temperature to rise uniformly and quickly during the heating process. The overflow path of the aerosol generated by heating and atomizing the aerosol-generating substrate strip 30 is short, so preheating is not necessary. It can be understood that the specific thickness and width of the aerosol-generating substrate strip 30 are not limited and can be set as needed to meet different requirements.

Furthermore, the aerosol-generating substrate strip 30 is made by mixing one or more of tobacco leaves, expanded tobacco stems, tobacco granules, tea leaves, or mint leaves, one or more of smoking agents such as propylene glycol, glycerol, or other polyols, as well as flavorings and fragrances to form a slurry, thus it can be heated and atomized to produce aerosols for inhalation. It can be understood that the material forming the aerosol-generating substrate strip 30 is not limited to this and can be set as needed to meet different requirements.

The atomization module 14 is in contact with one side of the aerosol-generating substrate strip 30, and the atomization module 14 can heat the aerosol-generating substrate strip 30 under the action of electrical energy from the power supply module to generate aerosols. Specifically, the atomization module 14 may be configured to include one or more of a resistance heating component, an infrared heating component, an electromagnetic heating component, or a plasma heating component. It can be understood that the heating principle of the atomization module 14 is not limited, and different heating methods can be used to heat and atomize the aerosol-generating substrate strip 30.

With continued reference to FIG. 2 and FIG. 3, in some embodiments, the drive module 13 includes a feed motor, a release reel 131, a retrieve reel 132, and a conveyor roller 133. In the conveying path of the aerosol-generating substrate strip 30, the conveyor roller 133 is located between the release reel 131 and the retrieve reel 132, the atomization module 14 is located between the release reel 131 and the conveyor roller 133, and the feed motor is connected to and drives the retrieve reel 132.

In this way, one end of the strip-shaped aerosol-generating substrate strip 30 is wound around the release reel 131. The other end of the aerosol-generating substrate strip 30 passes through the atomization module 14 and the conveyor roller 133 in sequence and is wound around the retrieve reel 132. The retrieve reel 132 rotates under the drive of the feed motor to continuously retrieve and wind up the aerosol-generating substrate strip 30, while the release reel 131 rotates synchronously to release the aerosol-generating substrate strip 30. The atomization module 14 can heat and atomize the aerosol-generating substrate strip 30 in contact with it, and the aerosol-generating substrate strip 30 after atomization is then retrieved by the retrieve reel 132.

In another embodiment, the feed motor may be connected to and drive the conveyor roller 133. The conveyor roller 133 rotates under the drive of the feed motor to move the aerosol-generating substrate strip 30 towards the retrieve reel 132, and the release reel 131 rotates synchronously to release the aerosol-generating substrate strip 30.

In some embodiments, the electronic atomizer further includes a retrieve cavity 30. The drive module 13 is located on one side of the retrieve cavity 30 and includes a feed motor, a release reel 135, a driving feed roller group 136, and a roller shaft group 137. The driving feed roller group 136 includes two driving feed rollers arranged at intervals, and a gap is formed between the outer surfaces of the two driving feed rollers for the aerosol-generating substrate strip 30 to pass through. The roller shaft group 137 includes two roller shafts arranged at intervals, and a gap is formed between the outer surfaces of the two roller shafts for the aerosol-generating substrate strip 30 to pass through.

In the conveying direction of the aerosol-generating substrate strip 30, the driving feed roller group 136 and the roller shaft group 137 are spaced apart, and the release reel 135 is located at one side of the driving feed roller group 136 away from the roller shaft group 137, and the atomization module 14 is located between the driving feed roller group 136 and the roller shaft group 137. The feed motor may be connected to and drive the driving feed roller group 136. The driving feed roller group 136 rotates under the drive of the feed motor to continuously drive the aerosol-generating substrate strip 30 to move forward for atomization. The aerosol-generating substrate strip 30 that has been heated and atomized by the atomization module 14 enters the retrieve cavity 30 for retrieval.

In another embodiment, the feed motor may alternatively be connected to and drive the driving feed roller group 136 and the roller shaft group 137, respectively. The driving feed roller group 136 and the roller shaft group 137 rotate under the drive of the feed motor to jointly drive the aerosol-generating substrate strip 30 to continuously move forward for atomization. The aerosol-generating substrate strip 30 that has been heated and atomized by the atomization module 14 enters the retrieve cavity 30 for retrieval.

In some embodiments, the electronic atomizer further includes a feed detection module 16. The feed detection module 16 is located on the moving path of the aerosol-generating substrate strip 30, preferably on the upstream of the atomization module 14. The feed detection module 16 is in communication connection with the main control module 11 and electrically connected to the power supply module, and is configured to detect a real-time released length of the aerosol-generating substrate strip 30 under the control of the main control module 11 and feed it back to the main control module 11, so as to accurately control the released length of the aerosol-generating substrate strip 30, preventing waste of the aerosol-generating substrate strip 30 while ensuring timely supply of the aerosol-generating substrate strip 30 to the atomization module 14.

Specifically, in an embodiment, the feed detection module 16 includes two rotating members 162, a sensing unit, and a detection unit. The two rotating members 162 are spaced apart, and a gap is formed between the outer cylindrical surfaces of the two rotating members 162 for the aerosol-generating substrate strip 30 to pass through. The two rotating members 162 are fitted on the two sides of the aerosol-generating substrate strip 30 in the thickness direction, respectively, and rotate under the drive of the aerosol-generating substrate strip 30. At least one rotating member 162 is provided with the sensing unit. The sensing unit may be a magnetic sensing element, a photoelectric sensing element, or a mechanical sensing element. The detection unit is located on one side of the rotating member 162 and includes a Hall sensor, a light shield, or a mechanical detection element that matches the sensing unit.

In this way, the detection unit can obtain the rotation angle or number of rotations of the rotating member 162 by counting the number of times the sensing unit passes through, and then obtain the real-time released length of the aerosol-generating substrate strip 30 based on the rotation angle or the number of rotations of the rotating member 162. It can be understood that the type of sensing unit arranged on the rotating member 162 is not limited and may also be other features or components that can be detected by the detection unit. Correspondingly, the type of detection unit is configured to match the type of sensing unit.

In some other embodiments, the feed motor is provided with a motor encoder, and a driving shaft of the feed motor directly abuts against the aerosol-generating substrate strip 30 to drive the aerosol-generating substrate strip 30 to move. The feed detection module 16 is connected to the motor encoder of the feed motor, and can obtain the real-time released length of the aerosol-generating substrate strip 30 by obtaining the number of rotations of the motor encoder.

In some other embodiments, detection markers may also be arranged on the aerosol-generating substrate strip 30. Multiple detection markers are spaced apart along the length direction of the aerosol-generating substrate strip 30. The detection markers include one or more of mechanical structural features such as horizontal lines, corrugations, grid patterns or holes, photoelectric features, magnetic features, or other detectable markers. Since the distance between each two spaced-apart detection markers is constant, the feed detection module 16 can obtain the real-time released length of the aerosol-generating substrate strip 30 based on the number of the markers or switching signals.

The start and stop signal input module 12 is mounted on the main housing, and may be a mechanical button, a touch button, a fingerprint button, a Hall sensor, or an airflow sensor that can sense airflow. It can be understood that the method of obtaining start command or stop command is not limited. The start and stop signal input module 12 can alternatively obtain the start signal or stop signal through voice recognition or gesture recognition.

In some embodiments, the electronic atomizer also includes a status indication module 17. The status indication module 17 is in communication connection with the main control module 11 and electrically connected to the power supply module, and is configured for indicating the operating status of the electronic atomizer under the control of the main control module 11. Specifically, the status indication module 17 may, under the control of the main control module 11, indicate a heating status, a driving status, and a puffing signal of the electronic atomizer to the user by outputting signals such as image, text, light, vibration, sound, so that the user can clearly know the operating status of the electronic atomizer.

In some embodiments, the electronic atomizer further includes an installation detection module 18. The installation detection module 18 is in communication connection with the main control module 11, and is configured to detect whether a substrate container exists in the electronic atomizer. In case of determining that no substrate container exists in the electronic atomizer, the installation detection module 18 can feed the detection result back to the main control module 11, so that the main control module 11 can prohibit, based on the detection result, the atomization module 14 from starting. In case of determining that a substrate container exists in the electronic atomizer, the installation detection module 18 can feed the detection result back to the main control module 11, so that the main control module 11 can start the atomization module 14 based on the detection result.

Specifically, the substrate container is provided with a sensing element such as a Hall sensor or a photoelectric sensor that matches the installation detection module 18. The installation detection module 18 may be a magnetic element that matches the Hall sensor or a light shield that matches the photoelectric sensor. The Hall sensor can determine whether a substrate container exists in the electronic atomizer by detecting whether a magnetic element exists. The photoelectric sensor can determine whether a substrate container exists in the electronic atomizer by detecting whether a light shield exists.

In some embodiments, the electronic atomizer also includes a residual detection module 19. The residual detection module 19 is in communication connection with the main control module 11 and is configured to detect the amount of unreleased aerosol-generating substrate strip 30, thereby reminding the user to check and replace the substrate container or the aerosol-generating substrate strip 30 timely. Specifically, the residual detection module 19 can detect changes in the stall current of the feed motor of the drive module 13, changes in the temperature curve of the atomization module 14, or the volume of the aerosol-generating substrate strip 30 to obtain the residual amount of the aerosol-generating substrate strip 30 and feed it back to the main control module 11, thereby outputting signals including but not limited to image, text, light, vibration, or sound to prompt the user to check and replace the aerosol-generating substrate strip 30.

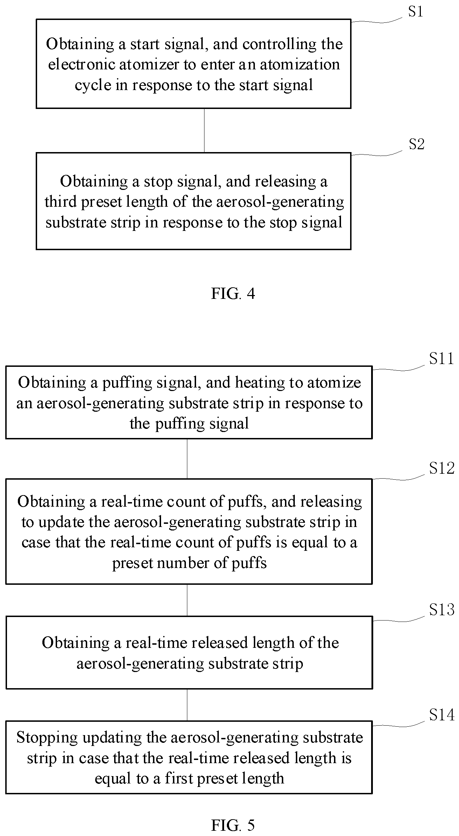

As shown in FIG. 4 and FIG. 5, an atomization control method for the above-mentioned electronic atomizer includes the following steps S1 and S2.

In step S1, a start signal is obtained, and the electronic atomizer is controlled to enter an atomization cycle in response to the start signal.

Specifically, the start signal is obtained by the start and stop signal input module 12. When a user presses the start and stop signal input module 12, the start and stop signal input module 12 may obtain the start signal and send it to the main control module 11. It can be understood that an approach with which the start-stop signal input module 12 obtains the start signal is not limited. The start and stop signal input module 12 may obtain the start signal by means of fingerprint recognition, touch recognition, airflow sensing, voice recognition, or gesture recognition.

Obtaining the start signal and controlling the electronic atomizer to enter the atomization cycle in response to the start signal in step S1 includes the following steps S11 to S14.

In step S11, a puffing signal is obtained, and the aerosol-generating substrate strip 30 is heated to be atomized in response to the puffing signal.

Specifically, the electronic atomizer is provided with an airflow sensor. The airflow sensor can be triggered to send a puffing signal to the main control module 11 when a user puffs through the airflow sensor. In response to the puffing signal, the control module 11 controls the atomization module 14 to heat the aerosol-generating substrate strip 30 to generate aerosols for the user to inhale.

In a specific embodiment, the atomization module 14 heats the aerosol-generating substrate strip 30 using a high-power rapid start. Each time the puffing signal is obtained, a heating duration of the atomization module 14 ranges 2.0 seconds to 3.0 seconds, preferably 2.0 seconds to 2.5 seconds. During the initial 0.2 seconds to 1 second, the atomization module 14 quickly heats up to a highest heating temperature, which may reach 350°C to 700°C.

In this way, when a user puffs the electronic atomizer, the atomization module 14 can immediately heat the aerosol-generating substrate strip 30 under the control of the main control module 11 to rapidly generate aerosols for the user to inhale, without waiting for preheating, improving the experience of using the electronic atomizer.

In step S12, a real-time count of puffs is obtained, and the aerosol-generating substrate strip is released for updating in case of determining that the real-time count of puffs is equal to a preset number of puffs.

Specifically, the main control module 11 obtains the real-time count of puffs and compares the real-time count of puffs with the preset number of puffs. In case of determining that the real-time count of puffs is equal to the preset number of puffs, the main control module 11 controls the drive module 13 to release the aerosol-generating substrate strip 30 to replace the aerosol-generating substrate strip 30 that has been atomized, preparing for a next atomization. It can be understood that the preset number of puffs may be one or more, and may be set as needed to fully utilize the aerosol-generating substrate strip 30.

In step S13, a real-time released length of the aerosol-generating substrate strip 30 is obtained.

Specifically, the feed detection module 16, under the control of the main control module 11, obtains the real-time released length of the aerosol-generating substrate strip 30 and feeds it back to the main control module 11.

In step S14, in case that the real-time released length is equal to a first preset length, updating of the aerosol-generating substrate strip 30 is stopped.

Specifically, the main control module 11 compares the real-time released length with the first preset length. In determining that the real-time released length of the aerosol-generating substrate strip 30 is equal to the first preset length, it is indicated that the updating of the aerosol-generating substrate strip 30 has been completed. In this case, in order to avoid waste of the aerosol-generating substrate strip 30, the main control module 11 controls the drive module 13 to stop releasing the aerosol-generating substrate strip 30. The first preset length is not less than an atomization length of the aerosol-generating substrate strip 30 within one atomization cycle.

It can be understood that the drive module 13 can periodically release to update the aerosol-generating substrate strip 30 under the control of the main control module 11, or continuously release the aerosol-generating substrate strip 30 under the control of the main control module 11 until the puffing ends. In the way of periodically releasing to update the aerosol-generating substrate strip 30, the updating may be performed after a waiting period following the heating of the aerosol-generating substrate strip 30 by the atomization module 14, facilitating the full atomization of the aerosol-generating substrate strip 30. According to a preferred embodiment, the waiting period for updating the aerosol-generating substrate strip after heating ranges from 0.5 seconds to 5 seconds.

In a specific embodiment, obtaining the real-time released length of the aerosol-generating substrate strip 30 in step S13 specifically includes the following steps S131 and S132.

In step S131, a rotation angle or a number of rotations of the rotating member 162 is obtained.

Specifically, the electronic atomizer includes a feed detection module 16, which includes a rotating member 162, a sensing unit, and a detection unit. The movement of the aerosol-generating substrate strip 30 drives the rotating member 162 to rotate synchronously. The detection unit can obtain the rotation angle or the number of rotations of the rotating member 162 by counting the times the sensing unit has passed by while following the rotation of the rotating member 162.

In step S132, the real-time released length of the aerosol-generating substrate strip 30 is obtained based on the rotation angle or the number of rotations of the rotating member 162.

Specifically, since the released length of the aerosol-generating substrate strip 30 corresponding to each unit angle of rotation of the rotating member 162 is constant, the feed detection module 16 can obtain the real-time released length of the aerosol-generating substrate strip 30 based on the rotation angle or the number of rotations of the rotating member and send the real-time released length to the main control module 11.

In another specific embodiment, obtaining the real-time released length of the aerosol-generating substrate strip 30 in step S13 specifically includes the following steps S133 and S134.

In step S133, a number of rotations of the motor encoder of the feed motor is obtained.

Specifically, a driving shaft of the feed motor of the drive module 13 abuts against the aerosol-generating substrate strip 30, and the feed detection module 16 is connected to the motor encoder of the feed motor to obtain the number of rotations of the motor encoder of the feed motor.

In step S134, the real-time released length of the aerosol-generating substrate strip 30 is obtained based on the number of rotations of the motor encoder.

Specifically, since the released length of the aerosol-generating substrate strip 30 corresponding to each unit angle of rotation of the driving shaft of the feed motor is constant, the feed detection module 16 can obtain the real-time released length of the aerosol-generating substrate strip 30 based on the number of rotations of the motor encoder and send the real-time released length to the main control module 11.

In a specific embodiment, obtaining the real-time released length of the aerosol-generating substrate strip 30 in step S13 specifically includes the following steps S135 to S136.

In step S135, a number of detection markers on the aerosol-generating substrate strip 30 is obtained.

Specifically, marking features are arranged on the aerosol-generating substrate strip 30. Multiple marking features are spaced apart along the length direction of the aerosol-generating substrate strip 30. As the aerosol-generating substrate strip 30 is released, the feed detection module 16 can obtain the number of the marking features.

In step S136, the real-time released length of the aerosol-generating substrate strip 30 is obtained based on the number of detection markers.

Specifically, since the distance between each two adjacent marking features is constant, the main control module 11 can calculate the real-time released length of the aerosol-generating substrate strip 30 based on the number of detection markers.

In some embodiments, the atomization control method further includes the following step:
obtaining a start signal and releasing a second preset length of aerosol-generating substrate strip 30 in response to the start signal.

Specifically, after the start and stop signal input module 12 obtains the start signal and feeds it back to the main control module 11, the main control module 11 controls the drive module 13 to release the second preset length of the aerosol-generating substrate strip 30 to replace the previously exposed aerosol-generating substrate strip 30, ensuring the consistency of the quality of the aerosol-generating substrate strip 30, thereby improving the consistency of each puff. It can be understood that the second preset length may be set according to the length of the exposed aerosol-generating substrate strip 30 to achieve the replacement of the aerosol-generating substrate strip 30.

In some embodiments, after obtaining the start signal and controlling the electronic atomizer to enter an atomization cycle in response to the start signal in step S1, the method further includes step S2.

In step S2, a stop signal is obtained, and a third preset length of the aerosol-generating substrate strip is released in response to the stop signal.

Specifically, the start signal is obtained by the start and stop signal input module 12. In a specific embodiment, when a user presses the start and stop signal input module 12, the start and stop signal input module 12 may obtain the stop signal and send it to the main control module 11. It is understood that an approach with which the start and stop signal input module 12 obtains the start signal is not limited. The start and stop signal input module 12 may obtain the start signal by means of fingerprint recognition, touch recognition, airflow sensing, voice recognition, or gesture recognition.

Furthermore, in some embodiments, the main control module 11 may alternatively obtain the stop signal by obtaining the operating time of the electronic atomizer, obtaining the operating temperature of the electronic atomizer, or determining the residual amount of the aerosol-generating substrate strip 30. In case that the operating time of the electronic atomizer is too long, the operating temperature is too high, or the residual amount of the aerosol-generating substrate strip 30 is too low, the main control module 11 may obtain a stop signal to stop the electronic atomizer in time, preventing the electronic atomizer from malfunctioning due to factors such as excessive operating time, excessive temperature, or empty burning.

When the drive module 13 obtains a stop signal, it controls the electronic atomizer to stop running, including stopping obtaining the count of puffs, controlling the atomization module 14 to stop heating the aerosol-generating substrate strip 30, controlling the drive module 13 and the feed detection module 16 to stop running, and controlling the status indication module 17 to output a signal to indicate the operating status of the electronic atomizer.

Specifically, since the aerosol-generating substrate strip 30 after atomization becomes thinner and is prone to breakage, before controlling the electronic atomizer to stop running, the main control module 11 controls the drive module 13 to release a first preset length of the aerosol-generating substrate strip 30 to replace the heated and atomized aerosol-generating substrate strip 30, thereby reducing the occurrence of breakage of the aerosol-generating substrate strip 30.

The above-mentioned atomization control method and atomization device utilize a strip-shaped aerosol-generating substrate strip 30 to achieve instant-on and instant-off functions. In other words, when a user puffs the electronic atomizer, the aerosol-generating substrate strip 30 can be quickly heated to produce aerosols, and when the user stops puffing, the heating can be stopped in time to avoid waste of the aerosol-generating substrate strip 30, thereby improving the utilization rate of the aerosol-generating substrate strip 30. In addition, by providing the feed detection module 16, the released length of the aerosol-generating substrate strip 30 can be detected in real time, so as to accurately control the released amount of the aerosol-generating substrate strip 30, improving the puffing consistency.

The technical features in the above embodiments may be randomly combined. For concise description, not all possible combinations of the technical features in the above embodiments are described. However, provided that the combinations of the technical features do not conflict with each other, all combinations of the technical features are considered as falling within the scope recorded in this specification.

The above-mentioned embodiments only illustrate several embodiments of the present disclosure, and the descriptions of which are relatively specific and detailed, but should not be construed as limitations to the scope of the present disclosure. It should be noted that, for those skilled in the art, variations and improvements can be made without departing from the concept of the present disclosure, which all belong to the protection scope of the present disclosure. Therefore, the protection scope of the present disclosure shall be subject to the appended claims.

## Claims

1. An atomization control method for an electronic atomizer, **characterized in that** the atomization control method comprises:
obtaining a puffing signal, and heating to atomize an aerosol-generating substrate strip in response to the puffing signal; and
obtaining a real-time count of puffs, and releasing to update the aerosol-generating substrate strip in case that the real-time count of puffs is equal to a preset number of puffs .

2. The atomization control method according to claim 1, further comprising:
obtaining a real-time released length of the aerosol-generating substrate strip; and
stopping updating the aerosol-generating substrate strip in case that the real-time released length is equal to a first preset length.

3. The atomization control method according to claim 2, wherein the electronic atomizer comprises a rotating member that rotates along with a release of the aerosol-generating substrate strip, and obtaining the real-time released length of the aerosol-generating substrate strip comprises:
obtaining a rotation angle or a number of rotations of the rotating member; and
obtaining the real-time released length of the aerosol-generating substrate strip based on the rotation angle or the number of rotations.

4. The atomization control method according to claim 2, wherein the electronic atomizer comprises a feed motor for releasing the aerosol-generating substrate strip, the feed motor comprising a motor encoder, and obtaining the real-time released length of the aerosol-generating substrate strip comprises:
obtaining a number of rotations of the motor encoder of the feed motor; and
obtaining the real-time released length of the aerosol-generating substrate strip based on the number of rotations of the motor encoder.

5. The atomization control method according to claim 2, wherein the aerosol-generating substrate strip is provided with detection markers arranged at intervals along a length direction of the aerosol-generating substrate strip (30), and obtaining the real-time released length of the aerosol-generating substrate strip comprises:
obtaining a number of detection markers on the aerosol-generating substrate strip; and
obtaining the real-time released length of the aerosol-generating substrate strip based on the number of detection markers.

6. The atomization control method according to claim 1, before obtaining the puffing signal and heating to atomize the aerosol-generating substrate strip in response to the puffing signal, the atomization control method further comprising:
obtaining a start signal, and releasing a second preset length of the aerosol-generating substrate strip in response to the start signal.

7. The atomization control method according to claim 1, further comprising:
obtaining a stop signal, and releasing a third preset length of the aerosol-generating substrate strip in response to the stop signal.

8. An electronic atomizer, **characterized in that** the electronic atomizer comprises:
a main control module;
a start and stop signal input modulewhich is in communication connection with the main control module and is configured to receive a start signal and feed the start signal back to the main control module;
a drive module in communication connection with the main control module, the drive module being configured to release, under control of the main control module (11), an aerosol-generating substrate strip for updating; and
an atomization module in communication connection with the main control module, the atomization module being configured to heat to atomize the aerosol-generating substrate strip under control of the main control module.

9. The electronic atomizer according to claim 8, further comprising a feed detection module, wherein the feed detection module is in communication connection with the main control module and is configured to obtain a real-time released length of the aerosol-generating substrate strip and feed the real-time released length back to the main control module.

10. The electronic atomizer according to claim 8, further comprising a status indication module, wherein the status indication module is in communication connection with the main control module and is configured to indicate an operating status of the electronic atomizer.

11. The electronic atomizer according to claim 8, further comprising a substrate container and an installation detection module, wherein the substrate container is configured to accommodate the aerosol-generating substrate strip, and the installation detection module is configured to detect whether the substrate container exists in the electronic atomizer.

12. The electronic atomizer according to claim 8, further comprising a residual detection module, wherein the residual detection module is configured to detect a residual amount of the aerosol-generating substrate strip.
